Europäisches Patentamt

European Patent Office

Office européen des brevets

(11) Publication number: **0 083 223**
**A2**

(12) # EUROPEAN PATENT APPLICATION

(21) Application number: 82306924.0

(22) Date of filing: 23.12.82

(51) Int. Cl.³: **B 01 F 17/42**
**C 11 D 3/00, A 61 K 7/06**

(30) Priority: 24.12.81 US 334248

(43) Date of publication of application:
06.07.83 Bulletin 83/27

(84) Designated Contracting States:
AT BE CH DE FR GB IT LI NL SE

(71) Applicant: LEISURE PRODUCTS CORPORATION
P.O. Box 684
Cascade Colorado 80809(US)

(72) Inventor: Church, Peter Kingsley
4585 Hagerman Road
Cascade Colorado 80809(US)

(74) Representative: Baillie, Iain Cameron et al,
c/o Ladas & Parry Isartorplatz 5
D-8000 München 2(DE)

(54) Oil and grease emulsification system.

(57) Compositions for use in the emulsification of oils and greases, and which contain soaps or synthetic surfactants as emulsifying agents, are synergistically improved for their intended use by addition of high molecular weight ethylene oxide based polymers.

EP 0 083 223 A2

## DESCRIPTION

## OIL AND GREASE EMULSIFICATION SYSTEM

### Technical Field

This invention relates to new and improved compositions for use in the emulsification of oils and greases, wherein certain water-soluble ethylene oxide based polymers, combined in a water based system with one or more soaps or synthetic surfactants, sometimes referred to hereinafter simply as "surfactants", are added to provide extremely rapid and efficient emulsification of such materials.

### Background Art

Small amounts of ethylene oxide based polymers, usually of relatively low molecular weight, have previously been added to window cleaner compositions containing alcohol, ammonia or analogous cleaning agents, and optionally containing very small proportions of certain surfactants to provide improved wicking action during toweling of the liquid from the glass. The compositions are ineffective as emulsifiers. More recently, extremely high molecular weight ethylene oxide based polymers, having a molecular weight of at least several hundred thousand, have been employed as antifogging agents in window cleaning compositions free of organic solvents but containing small proportions of surfactants. While these latter compositions may have some slight emulsifying properties they are so excessively unctuous and slippery that penetration and emulsification of oils and greases is attainable only with excessive mechanical working.

### Disclosure of Invention

The present invention may be broadly characterized as involving an emulsifier composition which, when made up with water to a total of 100 parts, contains as essential ingredients, and in effective amounts sufficient for said composition to provide by synergistic action easy and rapid emulsification of oils and greases, at least about 1-1/2 and preferably not more than about 9 percent of water soluble ethylene oxide based polymer having a molecular weight of at least about 12,000 but not more than about 80,000, and at least about 0.2 and preferably not more than about 10 percent of surfactant.

Best Mode of Carrying Out Invention

The ethylene oxide based polymers here under consideration consist of polyethylene glycol (PEG), alkyl derivatives of PEG such as methoxypolyethylene glycol (MPEG), cross linked PEG (PEGC), and copolymers such as polyoxyethylenepolyoxypropylene glycol (PEPG). Only those polymers which are water soluble and have molecular weights between about 12,000 and about 80,000 are practically and economically useful for the purposes of this invention. Some typical commercially available products which have been found useful are listed in the following Table.

Table 1

| "UCON" 75-H-90000 | linear PEPG copolymer of ethylene and propylene oxides; 12,000 mol. wt. |
| "CARBOWAX" 14,000 | straight chain linear PEG; 12,500-15,000 mol. wt. |
| "CARBOWAX" 20,000 | straight chain linear PEG; 18,000-19,000 mol. wt. |
| Polyethylene Glycol Compound 20M | low mol. wt. PEG cross linked with epoxide to a mol. wt. of 15,000-20,000 (PEGC). |
| XHS 1597:01 | normally liquid linear PEPG; 20,000-40,000 mol. wt. |

"CARBOWAX" and "UCON" are trademarks of, and Polyethylene Glycol Compound 20M is a product of, Union Carbide Corp.; XHS 1597:01 is a product of Dow Chemical Co.).

Typical soaps and synthetic surfactants which have been found highly effective in this invention are tabulated below.

Table 2

| Code | Product |
|------|---------|
| a, b | sodium and ammonium oleates |
| c | ammonium linoleate |
| d | ammonium linolenate |
| e, f, g | sodium, ammonium, triethanolamine lauryl sulfates |
| h | sodium-N-methyl-N-oleyl taurate |
| i | nonyl phenoxypoly(ethyleneoxy) ethanol |
| j | cocamidopropyl betaine |
| k | sodium (C14-16 olefin) sulfonate |
| l, m | sodium and ammonium lauryl ether sulfate (laureth sulfate) |
| n | sodium cocoyl isethionate |
| o | sodium methyl cocoyl taurate |
| p | N-methyl-N-(tall oil acyl)-taurine, sodium salt |
| q | nonyl phenoxypoly(ethyleneoxy)ethanol sulfate, ammonium salt |
| r | ammonium myreth sulfate |
| s | cetyl trimethyl ammonium chloride |

Other surfactants which of themselves are less highly effective are found to be useful in these emulsifier compositions either in somewhat larger amounts, or preferably in conjunction with small amounts of more highly effective surfactants, or with special preparation or handling. As an example of the latter, cocoyl sarcosine is water insoluble and cannot be directly incorporated, but can be colloidally dispersed in hot water and is then effective.

Excellent emulsification of oils and greases has been achieved with water based emulsifier compositions consisting essentially of about 4.5 - 9.0 percent of water soluble ethylene oxide based polymer of at least about 12,000 m.w. and about 0.2 - 0.3 percent of surfactant. The proportion of polymer may be significantly reduced, e.g. to about 1-1/2 percent or even lower, with the addition of extender materials such as cellulose ethers, mineral colloids, gelatin, or sodium polyacrylate. Methyl cellulose, hydroxypropyl cellulose, or hydroxypropyl methyl cellulose in amounts of about 1/4 to 1/2 percent, or mineral colloid in somewhat larger amounts of about 1 to 1-1/4 percent, make possible significant reduction in the amount of ethylene oxide based polymer required for full emulsification action. Larger amounts may also be used, e.g. for additional suspending or thickening action.

Many types of detergent builders have been found to be compatible and advantageous with the new emulsification systems. They include phosphates, citrates, carbonates, gluconates, tartrates and salts of ethylenediamine tetracetic acid (EDTA).

The compositions of this invention may be further modified for special end uses. For example, to prepare a shampoo or liquid hand soap the amount of surfactant may be greatly increased, e.g. up to about 10 percent, in order to obtain desirable sudsing action. Increased viscosity is obtained at appropriate pH levels with thickening agents, e.g. sodium or ammonium chloride, particularly in compositions containing PEGC's. Dry detergent products, e.g. soap powders or bar soaps, are improved by the addition of the ethylene oxide polymers, the necessary water being provided during use.

Formulations provided herein have been designed primarily for use at normal room temperatures. It will be understood that their effectiveness may be significantly increased where moderately elevated temperatures are involved, or conversely that the formulations may be modified to yield equivalent results at somewhat lower concentrations of active components.

In the formulas given, all proportions are by weight.

Emulsification testing was done using about 0.05 g. of a thick petroleum jelly rubbed on to the surface of a plate glass mirror to a diameter of approximately 7.5 cm. On this is deposited about 0.8 g. of the water based emulsifier composition which is then rubbed with the fingertips using a circular motion. An effective emulsifier rapidly results in a milky, uniform emulsion (OK). Less effective or ineffective materials cause the grease merely to separate into discrete chunks or coarse lumps, with little or no emulsification visible on inspection and with grease sticking to the fingers (NG).

### Example 1

| Material | A | B | C | D |
|---|---|---|---|---|
| sod. lauryl sulfate | .25 | .25 | .25 | .25 |
| PEGC 20,000 m.w. | 2.25 | 4.5 | 2.25 | 2.25 |
| hydroxypropyl methyl cellulose | - | - | .49 | - |
| mineral colloid | - | - | - | .98 |
| water to total of | 100 | 100 | 100 | 100 |
| Test Results: | NG | OK | OK | OK |

### Example 2

Similar tests were conducted on formulations containing 0.3 percent of soaps a, b or d (Table 2) with 4.75 percent of the PEGC, and others containing 0.25 percent of surfactants h, j, k, g, i or l with 4.5 percent of the PEGC. Complete emulsification resulted in all cases.

### Example 3

Complete emulsification is achieved with compositions containing 0.25 percent of cocamidopropyl betaine (Item j of Table 2) and 4.5 percent of PEGC 20,000 to which is added as detergent builders 1.5 percent of trisodium phosphate, 1 percent of ammonium citrate, 1 percent of sodium carbonate, or 0.4 percent of tetrasodium EDTA dihydrate. In this as in the other examples, water is added to a total of 100 percent (parts by weight) unless otherwise stated.

### Example 4

Water based compositions were prepared with 0.27 part of sodium-N-methyl-N-oleyl taurate (Item h of Table 2) and with various ethylene oxide based polymers in amounts just sufficient to obtain substantially the same speed and completeness of emulsification obtained for Example 1 B. Each composition was made up with water to a total of 100 parts. The polymers and amounts were as follows:

| polymer | PEPG | PEG | PEG | PEG | MPEG | MPEG |
|---|---|---|---|---|---|---|
| Mol. Wt. | 20,000-40,000 | 20,000 | 14,000 | 8,000 | 5,000 | 2,000 |
| parts required | 4.5 | 4.5 | 6.8 | 13.8 | 16 | 25 |

At polymer concentrations greater than about 7 or 8 parts the compositions become less desirable from a cost and efficiency aspect.

## Example 5

Effective emulsification has been achieved with compositions containing 0.20 part of sodium-N-methyl-N-oleyl taurate together with 2.0 parts of PEGC 20,000 m.w. and 0.49 part of methyl cellulose, or 1.47 parts of mineral colloid, and water to 100 parts. At the same level of surfactant, emulsification was obtained with a combination of 0.48 part of methyl cellulose and 4.5 parts of PEG 8,000 m.w. and with a combination of 1.36 parts of mineral colloid and 8.9 parts of PEG 8,000. A formulation containing only 0.17 part of the surfactant and 0.75 part of PEGC 20,000 with 0.50 part of hydroxylpropyl methyl cellulose was not fully effective although it did produce a substantial degree of visible emulsification.

## Example 6

Some illustrative examples of shampoo formulations.

| Material | 1 | 2 | 3 | 4 | 5 | 6 | 7 | 8 | 9 |
|---|---|---|---|---|---|---|---|---|---|
| sod. lauryl sulfate | 4.72 | 4.59 | 4.72 | - | 4.70 | 3.60 | 6.80 | - | 2.60 |
| sod. laureth sulfate | - | - | - | - | 4.10 | 3.10 | - | - | 2.27 |
| cocamidopropyl betaine | - | .825 | .85 | .90 | 1.02 | .75 | 1.23 | 1.14 | - |
| cocoyl sarcosine | - | .86 | - | - | - | - | - | - | - |
| sod. (C14-16 olefin) sulfonate | - | - | - | 4.96 | - | - | - | 5.72 | - |
| sod. lauryl sarcosinate | - | - | - | - | - | - | - | - | .33 |
| PEGC 20,000 | 5.66 | 5.50 | 2.83 | 4.00 | 3.00 | 3.00 | 3.00 | 4.00 | 4.00 |
| mineral colloid | 2.22 | 1.29 | 2.22 | - | - | - | - | - | - |
| lauramide DEA | .94 | - | .94 | .50 | .80 | .60 | .60 | .60 | 2.80 |
| thickener | - | - | - | NaCl | NaCl | NaCl | NaCl | NaCl | NaCl |

(water to 100)

These shampoo formulations, while containing much higher amounts of surfactants than the emulsifiers of the previous Examples, contain less than most if not all present day quality commercial liquid shampoos, and accordingly are much less likely to cause skin or eye irritation. In addition they produce a thicker lather of more substantial feel, and which more thoroughly penetrates between and separates the hairs. Less squeaking occurs during rinsing than is experienced with conventional shampoos; no creme rinse is necessary for long hair; shorter hair falls easily into place during blow drying; electrostatic effects are reduced or eliminated; and the hair is given a definite but natural sheen.

### Example 7

Some illustrative examples of liquid hand soap formulations.

| Material | 1 | 2 | 3 | 4 | 5 | 6 | 7 | 8 |
|---|---|---|---|---|---|---|---|---|
| sod. lauryl sulfate | 4.70 | 4.50 | - | - | 5.00 | 2.80 | - | 6.30 |
| sod. laureth sulfate | - | - | - | 2.40 | - | 2.32 | - | - |
| cocamidopropyl betaine | - | 1.98 | .90 | .54 | 1.29 | .90 | 1.62 | 1.62 |
| cocoyl sarcosine | 1.88 | - | - | - | - | - | - | - |
| sod. (C14-16 olefin) sulfonate | - | - | 4.96 | 2.72 | - | - | 6.24 | - |
| PEGC 20,000 | 5.60 | 5.30 | 3.00 | 3.00 | 6.00 | 4.00 | 3.00 | 3.00 |
| mineral colloid | 1.75 | 1.67 | - | - | - | - | - | - |
| methyl cellulose | - | - | .41 | - | - | - | - | - |
| lauramide DEA | - | - | .50 | .50 | - | 1.00 | .80 | .80 |
| thickener | - | - | NaCl | NaCl | NaCl | NaCl | NaCl | NaCl |
| water to 100 | | | | | | | | |

These hand soaps, which will also be seen to contain a far lower concentration of surfactants than most present-day commercial liquid soaps, have excellent sudsing qualities and lather well in the presence of various oils, greases and hand lotions rubbed into the hands before use.

### Example 8

Some illustrative examples of cleansing powders and bar soaps.

| Material | 1 | 2 | 3 | 4 |
|---|---|---|---|---|
| "BON AMI" cleanser | 94 | - | - | - |
| "DIAL" bar soap | - | 94 | - | - |
| "IVORY" bar soap | - | - | 95 | - |
| sodium oleate | - | - | - | 8 |
| PEGC 20,000 | 6 | 6 | 5 | 6 |
| $CaCO_3$ (-100 mesh) | - | - | - | 80 |
| $Na_2CO_3.10\ H_2O$ | - | - | - | 6 |

"BON AMI" is a cleanser in powder form containing a detergent, mild abrasive powder, sodium carbonate and bleach, commercially available from Faultless Starch/Bon Ami Company, Kansas City, Missouri 64101.

"DIAL" is a soap in bar form commercially available from Armour-Dial Inc., Phoenix, Arizona. "IVORY" is a soap in bar form commercially available from Proctor and Gamble Co., Cincinnati, Ohio.

The materials for samples 2 and 3 of Example 8 were mixed in powdered or shredded form with a minimum of water, pressed into bar form, and dried before testing. Samples 1 and 4 were mixed and remained in powder form. All samples showed increased effectiveness in emulsifying and removing various types of oil and grease from various test surfaces as compared with the same products from which the polymer was omitted.

The addition of small quantities of high molecular weight ethylene oxide based polymers to many well-known commercial liquid cleaners has likewise been found to result in a surprising improvement in the ability of such compositions to emulsify and remove oily and greasy contaminants from a variety of surfaces. Since such cleaners contain moderate to high concentrations of surfactants in a water based concentrate it will be readily seen that with the addition of the polymer they are brought within the ambit of the present invention.

- 1 -

## CLAIMS

1. An emulsifier composition which, when made up with water to a total of 100 parts, contains, as essential ingredients and in effective amounts sufficient to provide easy and rapid emulsification of petroleum jelly in the test procedure described in the specification, at least about 1.5 percent of water soluble ethylene oxide based polymer having a molecular weight of about 12,000 to about 80,000 and at least about 0.2 percent of surfactant.

2. Composition of claim 1 wherein said polymer is between 1.5 and 9 percent and said surfactant is between 0.2 and 10 percent.

3. Composition of claim 1 wherein is included as an extender a water soluble cellulose ether, water dispersible mineral colloid, gelatin, or sodium polyacrylate.

4. Composition of claim 1 wherein is included a detergent builder.

5. Composition of claim 1 wherein said polymer is crosslinked polyethylene glycol.

6. Composition of claim 1 wherein said polymer is a normally liquid copolymer of ethylene oxide and propylene oxide.

7. Composition of claim 5 having good sudsing and foaming characteristics and wherein said polymer is present in amount between about 3 and about 7 percent and said surfactant is present in amount between about 4.5 and about 10 percent.

8. Composition of claim 5 wherein said polymer is crosslinked polyethylene glycol having a molecular weight of about 20,000 and wherein said composition contains an alkali halide thickening agent.

9. Composition of claim 5 wherein is included an extender.